# EUROPÄISCHE PATENTSCHRIFT

(11) **EP 0 740 951 B1**
(45) Veröffentlichungstag und Bekanntmachung des Hinweises auf die Patenterteilung: **16.08.2001**
(21) Anmeldenummer: 96103548.2
(22) Anmeldetag: 07.03.1996
(51) Int. Cl.: B01D 67/00, B01D 71/68, B01D 71/56

(54) **Mit Hitze sterilisierbare Membran**
Sterilisable membrane by heat treatment
Membrane stérilisable par chaleur

(30) Priorität: 20.04.1995 DE 19514540
(43) Veröffentlichungstag der Anmeldung: 06.11.1996
(73) Patentinhaber: GAMBRO DIALYSATOREN GMBH & CO. KG, D-72379 Hechingen (DE)
(72) Erfinder: Bell, Carl-Martin, Dr., 72379 Hechingen (DE); Buck, Reinhold, Dipl.-Ing., 88422 Alleshausen (DE); Thomé, Barbara, Dipl.-Ing., 70794 Filderstadt (DE); Storr, Markus, Dr., 72379 Hechingen (DE); Göhl, Hermann, Dipl.-Ing., 72406 Bisingen (DE)
(74) Vertreter: Weber, Dieter, Dr.

(56) Entgegenhaltungen:
- EP-A- 0 082 433
- EP-A- 0 215 549
- EP-A- 0 368 447
- EP-A- 0 382 009
- EP-A- 0 604 882
- EP-B- 0 228 072
- WO-A-89/04200
- DE-A- 4 219 218
- DE-A- 4 232 082
- PATENT ABSTRACTS OF JAPAN vol. 7, no. 268 (C-197), 30.November 1983 & JP-A-58 150403 (NITTO DENKI KOGYO KK), 7.September 1983,
- PATENT ABSTRACTS OF JAPAN vol. 11, no. 398 (C-466), 25.Dezember 1987 & JP-A-62 160109 (FUJI PHOTO FILM CO LTD), 16.Juli 1987,
- PATENT ABSTRACTS OF JAPAN vol. 950, no. 5 & JP-A-07 116484 (TOYO ROSHI KAISHA LTD), 9.Mai 1995,
- PATENT ABSTRACTS OF JAPAN vol. 11, no. 163 (C-424), 26.Mai 1987 & JP-A-61 293502 (SUMITOMO BAKELITE CO LTD), 24.Dezember 1986,

## Beschreibung

Die Erfindung betrifft permselektive Membranen, die in Verbindung mit bestimmten medizinischen Behandlungen eingesetzt werden können, wie für die Hämodialyse, Hämofiltration, Hämodiafiltration, Plasmapherese oder Immunotherapie. Sie können auch für eine Dialyse oder Ultrafiltration allgemein, wie beispielsweise zur Reinigung von Wasser, benutzt werden.

Ein wichtiges Erfordernis für die Anwendbarkeit von Membranen in der Medizin, wie bei der Blutreinigungstherapie, besteht darin, daß die Membranen sterilisiert werden können. Aus Sicherheits- und Umweltschutzgründen sowie im Hinblick auf Nebenprodukte und toxische Rückstände ist die Wasserdampfsterilisierung oder Sterilisierung mit trockener Hitze gegenüber einer Sterilisierung mit Ethylenoxidgas, Plasmaverfahren, z. B. mit Wasserstoffperoxid oder durch Bestrahlung, wie mit γ-Strahlen, bevorzugt.

Ein Sterilisieren mit Wasserdampf erfolgt gewöhnlich durch Behandlung der Membraneinrichtung während etwa 20 min mit gesättigtem Wasserdampf bei einer Temperatur von etwa 120 °C. Sterilisieren mit trockener Hitze wird während 90 min bei 180 °C (DAB 1992) durchgeführt. Diese Sterilisationsbedingungen beschränken die'Anzahl der hierfür einsetzbaren synthetischen Polymere. So können beispielsweise Dialysemembranen aus Polyacrylnitril (US-PS 4 545 910), Cellulosetriacetat (US-PS 4 276 137) und teilaromatischem amorphem Polyamid (EP-OS 0 305 787) nicht mit Wasserdampf sterilisiert werden, da bei den Bedingungen der Wasserdampfsterilisation eine irreversible nachteilige Veränderung der Porenstruktur auftritt. Hydrolyseempfindliche Polymere, wie Poly-(carbonatblockethylenoxid) (US-PS 4 069 151), können auch nicht mit Wasserdampf sterilisiert werden, da das Polymer dabei abgebaut würde, was zu Brüchen und Löchern in der Membran führen würde. Außerdem werden diese Membranen mit hydrophilisierenden Mitteln imprägniert, wie mit Polyvinylpyrrolidon, Glycerin oder Polyethylenglycol (EP-OS 0 228 072), welche die Instabilität der Membranstruktur durch Erhitzen infolge ihres Erweichungseffektes weiter erhöhen.

Andererseits haben gerade solche Dialysemembranen aus diesen Polymeren ungewöhnlich gute Hämokompatibilität im Vergleich mit Dialysemembranen vom Cellulosetyp (siehe "Polyamide - the Evolution of a Synthetic Membrane for Renal Therapy", Herausgeber S. Shaldon, K. M. Koch in Contributions to Nephrology, Band 96, Karger [1992], E. Wegmüller, A. Montandon "Biocompatibility of Different Hemodialysis Membranes, Activation of Complement and Leukopenia", Inte. J. Artif. Organs 9, 85 [1986], R. M. Hakim et al "Complement Activation and Hypersensitivity Reactions to Dialysis Membranes", New Engl. J. Med. 311, 878 [1984], H. Schiffl "Biocompatible Membranes in Acute Renal Failure, Prospective Casecontrolled Study", Lancet 344, 570 [1994] und R. M. Hakim et al "Effect of the Dialysis Membrane in the Treatment of Patients with Acute Renal Failure, New Engl. J. Med. 311, 1338 [1994]).

Weiterhin weist im besonderen teilaromatisches, amorphes Polyamid ein besonders hohes Endotoxin- bzw. Pyrogenrückhaltevermögen auf. Lonnemann et al diskutieren in "Poyrogen Retention by the Polyamide Membrane" in "Polyamide - The Evolution of a Synthetic Membrane for Renal Therapy", ed. by S. Shaldon, K. M. Koch, Contributions to Nephrology 96, 47 (1992) die hohe Effizienz der asymmetrischen Polyamidmembrane bezüglich des Endotoxinrückhaltevermögens und messen der Interaktion der pyrogenen bakteriellen Substanzen mit den hydrophoben Domänen der Membran, die durch Polyamid gebildet werden, eine entscheidende Bedeutung zu.

Die EP-OS 0 604 882 beschreibt eine semipermeable Membran aus sulfoniertem aromatischem Polyetherketon und vollaromatischem Polyamid gegebenenfalls unter Zusatz von Polyvinylpyrrolidon. Die beiden Polymere sind unter den Bedingungen der Wasserdampfsterilisierung thermisch stabil, so daß es nicht überraschend ist, daß eine solche Membran mit Wasserdampf sterilisierbar ist.

Die der Erfindung zugrundeliegende Aufgabe besteht somit darin, unter Mitverwendung eines bei üblichen Bedingungen der Sterilisierung mit Wasserdampf oder trockener Hitze, d. h. bei gesättigtem Wasserdampf und einer Temperatur von 120 °C, instabilen Polymers, wie z. B. Polyamid oder Polyvinylpyrrolidon, eine mit Hitze, insbesondere mit Wasserdampf sterilisierbare Membran zu erhalten. Diese Aufgabe wird mit einer Membran mit den in Anspruch 1 angegebenen Merkmalen gelöst.

Es ist überraschend, daß die erfindungsgemäße Membran mit Wasserdampf oder trockener Hitze sterilisierbar ist, obwohl sie zum Teil aus einem Polymer oder Polymeranteil besteht, das bzw. der als solches bzw. solcher unter den üblichen Bedingungen der Wasserdampfsterilisierung nicht stabil ist und sich in seiner Struktur nachteilig verändert. Nach dem Prinzip des schwächsten Kettengliedes wäre zu erwarten gewesen, daß die erfindungsgemäßen Membranen bei der Sterilisierung mit Wasserdampf in den Bereichen des an sich instabilen Polymers abgebaut werden und die Membran dadurch insgesamt unbrauchbar wird. Überraschenderweise wird aber das an sich unter den Bedingungen der Wasserdampfsterilisierung oder der Sterilisierung bei trockener Hitze instabile Polymer stabil, wenn man ein bei den angegebenen Bedingungen stabiles Polymer unter Anwendung einer Kompatibilisierungsmethode zumischt oder aber an das instabile Polymer kovalent bindet.

Zweckmäßig besteht die erfindungsgemäße Membran aus 1 bis 30 Gew.-% des wenigstens einen bei den Bedingungen der genannten Sterilisierung instabilen Polymers und 70 bis 99 Gew.-% des wenigstens einen bei den Bedingungen der genannten Sterilisierung stabilen Polymers. Besonders günstig sind Membranen aus 4 bis 12 Gew.-% des ersteren und 88 bis 96 Gew.-% des letzteren Polymeren.

Im folgenden wird der Einfachheit halber das unter den Bedingungen der genannten Sterilisierung instabile Polymer als "thermisch instabiles Polymer" und das bei den nämlichen Bedingungen stabile Polymer als "thermisch stabiles Polymer" bezeichnet.

Als thermisch stabiles Polymer wird zweckmäßig ein solches mit einer Glasübergangstemperatur höher als 200 °C verwendet, wie zweckmäßig ein Polyethersulfon, Polysulfon, Polyetherketon oder Polyaramid. Polyethersulfone und Polysulfone sind bevorzugt, insbesondere Polyethersulfone.

Die thermisch instabilen Polymere sind zweckmäßig teilaromatische amorphe Polyamide, Polycarbonatether, Polyacrylnitril und Cellulosetriacetat. Eine besonders zweckmäßige Kombination, sei es in Form einer kompatibilisierten Mischung oder kovalent aneinander gebundener Polymere, sind teilaromatische amorphe Polyamide als thermisch instabile Polymere mit Polyethersulfonen als thermisch stabile Polymere. Polyethersulfone besitzen beispielsweise sich wiederholende Struktureinheiten der Formeln, die auf Seite 2 der DE-OS 4 219 218 angegeben sind.

Lösungen zweier Polymere, die als solche nicht miteinander mischbar sind, trennen sich häufig in zwei Phasen und ergeben eine Emulsion, in welcher das löslichere Polymer das weniger lösliche Polymer "aussalzt". Durch Verwendung oberflächenaktiver Substanzen oder anderer amphiphiler Stoffe, wie von Blockcopolymeren, die Bereiche unterschiedlicher Polaritäten aufweisen, kann die Größe der Tröpfchen der Emulsion reguliert und stabilisiert werden. So kann die Kompatibilisierung der beiden an sich nicht miteinander mischbaren oder kompatiblen hydrophoben Polymere mit Hilfe eines amphiphilen Mittels erzielt werden. Dieses wird zweckmäßig in einer Menge von 5 bis 10 Gew.-%, bezogen auf das Gewicht der gesamten verwendeten Polymeren, benutzt. Mit solchen amphiphilen Mitteln wird einerseits die Oberflächenspannung reduziert und andererseits ein Gemisch mit höherem Dispersitätsgrad und erhöhter Adhäsion gebildet (siehe R. Fayl, R. Jerome, ACS Symposium Ser. 395, 38 ([1989]). Durch amphiphile Blockcopolymere werden starke H-Bindungen an einem Block und Bindungen durch van der Waals-Kräfte an dem anderen Block ausgebildet, was zu einer Kompatibilisierung der beiden an sich nicht mischbaren hydrophoben Polymeren führt, so daß Gemische der beiden Polymeren im Molekularbereich und/oder Nanometerteilchenbereich entstehen. Hierfür geeignete amphiphile Blockcopolymere sind beispielsweise Poly-(styrol-b-ethylenoxide), in welchen der Polyethylenoxidrest die Wasserstoffbindung des Polyamids schwächt und der Polystyrolrest eine Wechselwirkung zu den Polyethersulfonmolekülen aufbaut.

Stattdessen kann die Kompatibilisierung der beiden Polymertypen auch mit anderen Substanzen erfolgen, die einerseits starke H-Bindungsstellen und andererseits hydrophobe oder van der Waals-Bindungsstellen haben, wie mit Ethanol und anderen Alkoholen, Harnstoff, Polyvinylpyrrolidon, ∈-Caprolactam, organischen Säuren oder Polyethylenglycol.

Weiterhin kann eine Kompatibilisierung durch bifunktionelle reaktive Verbindungen, wie Bisoxazoline oder Bismaleinsäureimidderivate, erfolgen, welche die intramolekularen und intermolekularen Kräfte zwischen den beiden Polymertypen, wie Polyamid und Polyethersulfon, schwächen und eine intermolekulare Adhäsion von Makromolekülen der beiden an sich nicht mischbaren Polymertypen ergeben.

C. Mees et al beschreiben in Journal of Polymer Science 32 (16, 3171 [1994]) die Synthese von aminendgruppenmodifizierten Polyethersulfonen durch Umetherungsreaktion während der Polykondensation in Gegenwart von m-Aminophenol. Anschließend verkappen sie dieses Polyethersulfon mit Maleinsäureanhydrid und erhalten so anhydridendgruppenmodifiziertes Polyethersulfon mit Glasübergangstemperaturen von ca. 270 °C.

S. J. Park beschreibt in "Polymer Bulletin" 29 (5), 477 (1992) eine Copolykondensation mit aminogruppenhaltigen Monomeren in Seitengruppen zur Synthese von aminfunktionalen Polyethersulfonen. Mit dieser Methode ist der Aminierungsgrad durch den Copolymeranteil einstellbar.

Polyamide, z. B. Trogamid T® (Hüls), haben je nach Monomerverhältnis endständnige Aminogruppen bzw. Carboxylgruppen.

Eine Kompatibilisierung wird durch kovalente Anbindung des Polyamids mit den beschriebenen, funktionalisierten Polyethersulfonmodifikationen nach folgendem Reaktionsschema erhalten:
a) PESU-NH₂ (aminoendgruppenfunktionalisiertes Polyethersulfon) 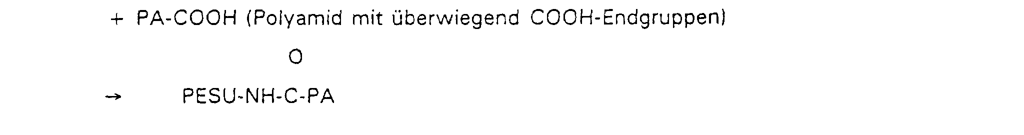
b) PESU (aminoseitengruppenfunktionalisiertes Polyehtersulfon) 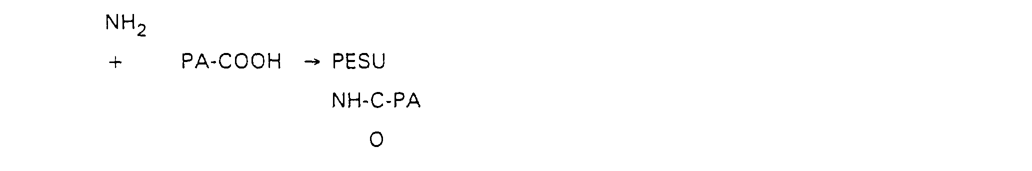
c) PESU-NH-AH (anhydridendgruppenfunktionalisiertes Polyethersulfon)
   + PA-NH₂ (Polyamid mit überwiegend NH₂-Gruppen endgruppenfunktionalisiert)
   → PESU-NH-A-NH-PA
d)

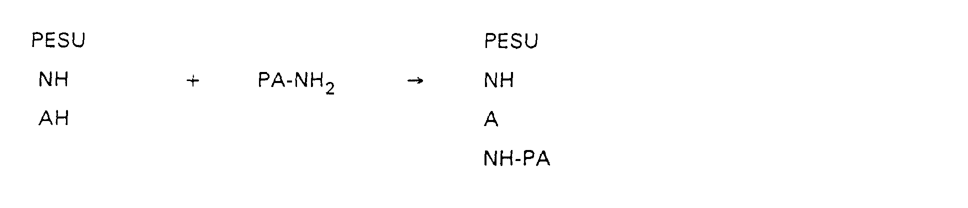

Die Reaktionen können in der Schmelze, im Extruder oder in einem inerten Lösungsmittel bei erhöhter Temperatur durchgeführt werden. Anschließend werden die kompatibilierten Coppolymere aus Lösung zu Membranen gesponnen.

Noch eine andere Art der Kompatibilisierung besteht darin, in die thermisch stabilen Polymeren, wie Polyethersulfon, Gruppen einzuführen, die starke H-Bindungen bilden, welche eine Wechselwirkung mit dem thermisch instabilen Polymer, wie Polyamid, erlauben. Dies kann man erreichen, indem man die thermisch stabilen Polymere sulfoniert, wobei der Sulfonierungsgrad nach dem erwünschten Mischungsgrad und der erwünschten Teilchengröße eingestellt werden kann. Solche sulfonierten Polymere, speziell Polyethersulfone und Polysulfone, sind in der DE-OS 4 219 218 und der dort zitierten Literatur beschrieben.

Eine weitere Methode zur Kompatibilisierung der beiden hier verwendeten Polymertypen besteht in der Verwendung anorganischer Füllstoffe, wie in der EP-OS 0 398 093 beschrieben ist. Solche anorganischen Füllstoffe sind beispielsweise TiO₂, BaSO₄ oder SiO₂ in der Form feiner Mikro- oder Nanometerteilchen, die in aprotischen dipolaren Lösungsmitteln eingesetzt werden. Zusätzlich können dabei oberflächenaktive Stoffe, wie ionische oder nichtionische Detergentien, benutzt werden. Andererseits kann man in Salzlösungen, wie solchen von Lithiumchlorid oder Calciumchlorid, die intermolekularen und intramolekularen H-Bindungen der thermisch instabilen Makromoleküle abbauen und so starke Wechselwirkungen beispielsweise zwischen Sulfon- und Carbonamidgruppen des Polyamids erzeugen, was zu einer Mischbarkeit im Nanometerbereich bis molekularen Bereich führen kann.

Vorzugsweise bekommt man bei der Kompatibilisierung Gemische der thermisch instabilen und thermisch stabilen Polymere mit Domänengrößen von 0,01 bis 1 *µ*m.

Arakawa et al beschreiben die Relevanz der Domänengröße in Artificial Organs 16 (2), 146 (1992) bezüglich der Thrombogenität bei Poly(acrylnitril-coethyloxid)-Hohlfasermembrandialysatoren und finden, daß Domänengrößen unter 100 nm signifikante Verbesserungen bezüglich den Blutgerinnungsparametern ergeben.

Statt einer Kompatibilisierung der beiden Polymertypen zur Erzeugung von Gemischen der beiden Polymertypen können diese auch zur Vermeidung einer Entmischung kovalent an ihrer Grenzfläche aneinander gebunden werden. Dies erreicht man durch Grenzflächenreaktion der funktionalisierten Polymere (d. h. über deren Endgruppen) mit einem reaktiven Kompatibilisierungsmittel. Beispielsweise kann Polystyrol mit Polyamid mit Hilfe des Kompatibilisierungsmittels Poly-(styrol)-comaleinsäureanhydrid oder Polyethylen mit Polyamid unter Verwendung des Kompatibilisierungsmittels Poly-(ethylenmaleinsäureanhydrid) verbunden werden.

Die erfindungsgemäßen Membranen können in üblicher Weise Bogenform oder Hohlfaserform haben und sind für die Dialyse, die Blutreinigungstherapie und/oder Ultrafiltration gut geeignet.

### Beispiele

### Beispiel 1

In einem mit Thermostat versehenen Rundkolben mit einem Polytetrafluorethylen-Rührer wurde ein teilaromatisches Polyamid (Trogamid T, Hüls) in den in der Tabelle angegebenen Gewichtsprozentangaben unter heftigem Rühren in N-Methylpyrrolidon aufgelöst. Nach vollständigem Lösen wurde das in der Tabelle angegebene Kompatibilisierungsmittel zugegeben und vollständig aufgelöst. Danach wurde Polyethersulfon oder modifiziertes Polyethersulfon zugegeben und gelöst, und in der letzten Stufe wurde gegebenenfalls zusätzliches Additiv zugegeben, um die Membranleistung einzustellen.

Die erhaltene Polymerlösung wurde durch ein Filter mit Porenöffnungen von 2 *µ*m filtriert und entgast, und die Viskosität bei 22 °C wurde gemessen. Aus der Polymerlösung wurden in üblicher Weise Hohlfasermembranen hergestellt, indem die Polymerlösung durch eine Ringspaltdüse bei der in der Tabelle angegebenen Temperatur extrudiert und mit der in der Tabelle angegebenen Mittellösung ausgefällt wurde. Die Hohlfaser wurde aus der Düse mit einer Geschwindigkeit von 40 bis 80 m/min abgezogen und durch eine Reihe von Spülbädern geführt. Sodann wurden die Hohlfasern aufgewickelt, zu Bündeln von jeweils hundert geschnitten, bei 45 °C getrocknet, in Polyurethan eingegossen, mit Wasserdampf sterilisiert und gekennzeichnet. Die Leistung der Hohlfasermembranen ist in der Tabelle angegeben. In den Spalten "Polymerlösung" und "Mittellösung" bedeuten die Zahlenangaben jeweils den Prozentgehalt. In der Tabelle bedeuten die Abkürzungen folgendes:
- Lpl:: Hydraulische Permeabilität, gemessen für reines Wasser (10⁻⁴ cm/s/bar) gemäß "Evaluation of Hemodialysis and Dialysis Membranes", NIH-Veröffentlichung 77-1294 [1977].
- Lp(Alb):: Hydraulische Permeabilität für eine 6 %ige Rinderalbuminlösung (10⁻⁴ cm/s/bar), gemessen nach der in "Evaluation of Hemodialysis and Dialysis Mebranes", NIH-Veröffentlichung 77-1294 [1977] beschriebenen Methode.
- Lp2:: Hydraulische Permeabilität für reines Wasser nach der Lp-(Alb)-Messung (10⁻⁴ cm/s/bar) gemessen gemäß "Evaluation of Hemodialysis and Dialysis Membranes", NIH-Veröffentlichung 77-1294 [1977].
- P(Cl):: Diffusionspermeabilität für eine 0,9 %ige NaCl-Lösung (10⁻⁴ cm/sec), gemessen gemäß "Evaluation of Hemodialysis and Dialysis Membranes", NIH-Veröffentlichung 77-1294 [1977].
- Sk(Myo):: Siebkoeffizient für eine 0,002 %ige Myoglobinlösung nach 15 min (%), gemessen gemäß DIN 58 353, Teile 2 C 3, 1988.
- Sk(Alb):: Siebkoeffizient einer 6 %igen Rinderalbuminlösung nach 15 min (%), gemessen nach DIN 58 353, Teil 2 C 3, 1988.

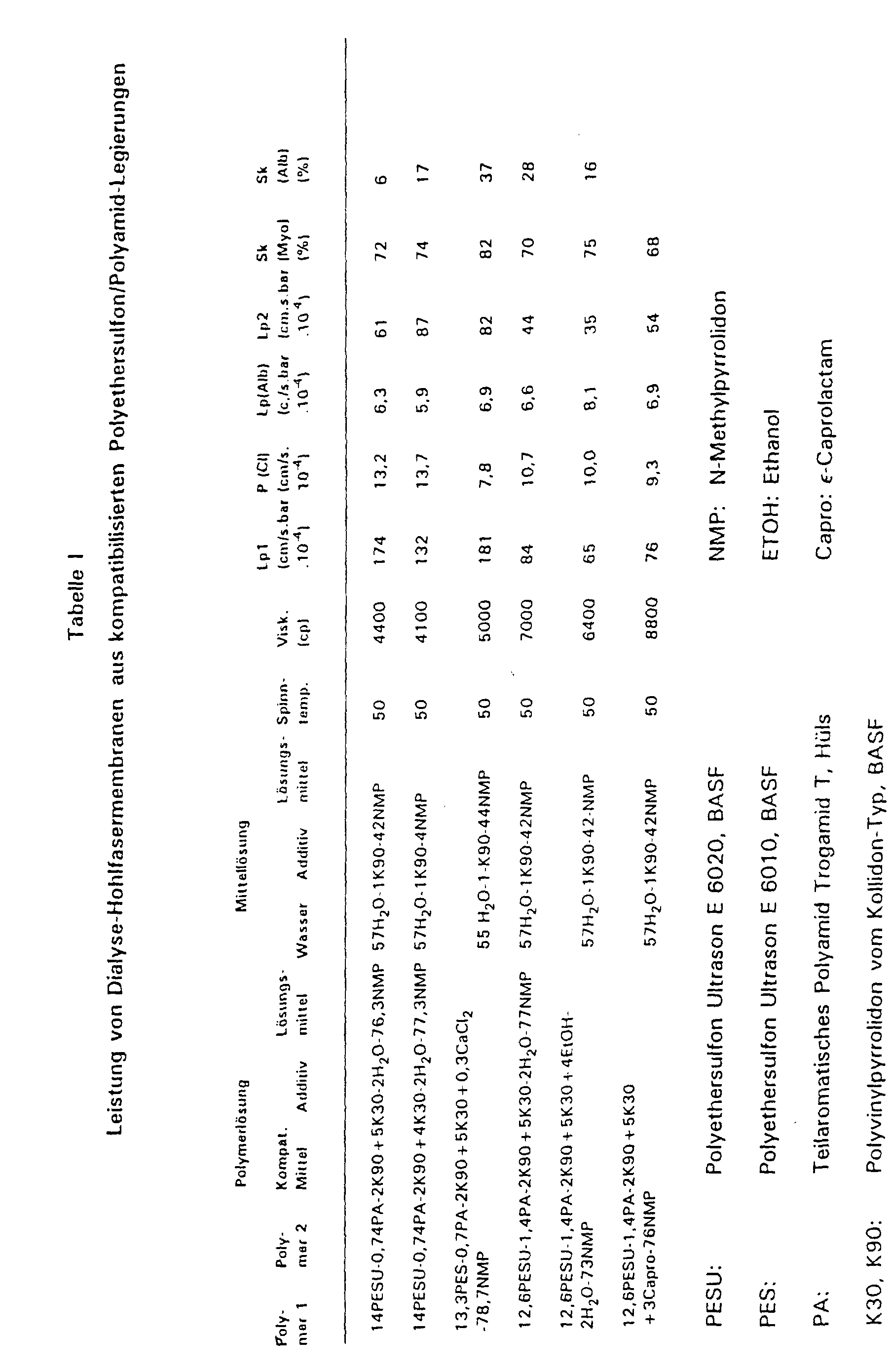

### Beispiel 2

Polyethersulfon wurde sulfoniert entsprechend DE-OS 4 219 218 mit 0,7 mmol/g Sulfonierungsgrad. Eine Polymerlösung mit der Zusammensetzung 13,3 Gew.-% sulfoniertes Polyethersulfon mit 0,7 Gew.-% Polyamid Trogamid T® (Hüls) ergibt in NMP klare Lösung, während die entsprechende Lösung aus demselben nichtsulfonierten Polyethersulfon eine trübe, nicht homogen mischbare Polymerlösung ergibt. Die Permeabilität von aus diesen Polymerlösungen hergestellten, nassen Flachmembranen in Wasser gefällt entsprechend obig zitiertem Patent sind in folgender Tabelle dargestellt:

| **Lösungsmittelzusammensetzung** | **L**_{**p**} **(H**_{**2**}**O) [10**^{**-4**} **cm/s · bar]** | **P (Cl) [10**^{**-4**} **cm/s]** |
|---|---|---|
| 13,3 % sulfononiertes Polyethersulfon + 0,7 % Trogamid T | 300 | 12,2 |
| 13,3 % Polyethersulfon + 0,7 % Trogamid T | 240 | <0,5 |

Durch Sulfonierung der hydrophoben Komponente läßt sich eine Kompatibilisierung mit der hydrophilen Kommponente erreichen.

### Beispiel 3

In einem mit Thermostat versehenen Rundkolben mit einem Polytetrafluorethylen-Rührer wurde das in Beispiel 1 verwendete teilaromatische Polyamid (Trogamid T, Hüls) in den in der Tabelle angegebenen Gewichtsprozentangaben unter heftigem Rühren bei 60 °C in N-Methylpyrrolidon gelöst. Nach vollständigem Lösen wurde das in der Tabelle angegebene Kompatibilisierungsmittel zugegeben und vollständig aufgelöst. Danach wurde Polyethersulfon und zusätzliches Additiv zugegeben und nach und nach aufgelöst.

Die erhaltene Polymerlösung wurde durch ein Filter mit Porenöffnungen von 2 *µ*m filtriet und entgast und durch die Viskositätsmessung bei 22 °C gekennzeichnet. Um Hohlfasern herzustellen, wurde die Polymerlösung durch eine Ringspaltdüse bei der in der Tabelle angegebenen Temperatur extrudiert und mit der in der Tabelle angegebenen Mittellösung ausgefällt. Die Hohlfaser wurde aus der Düse mit einer Geschwindigkeit von 40 bis 80 m/min abgezogen und durch eine Reihe von Spülbädern geführt. Sodann wurde die Hohlfaser aufgewickelt, zu Bündeln von jeweils 100 geschnitten, bei 45 °C getrocknet, in Polyurethan eingegossen, mit Wasserdampf sterilisiert und gekennzeichnet. Die Leistung der Hohlfasermembranen ist in der Tabelle angegeben. Wie die Angaben in der Tabelle zeigen, sind die erhaltenen Membranen für die Verwendung bei Mittelflußhämodialyse geeignet. Die Abkürzungen in diesem Beispiel haben die gleiche Bedeutung wie im Beispiel 1.

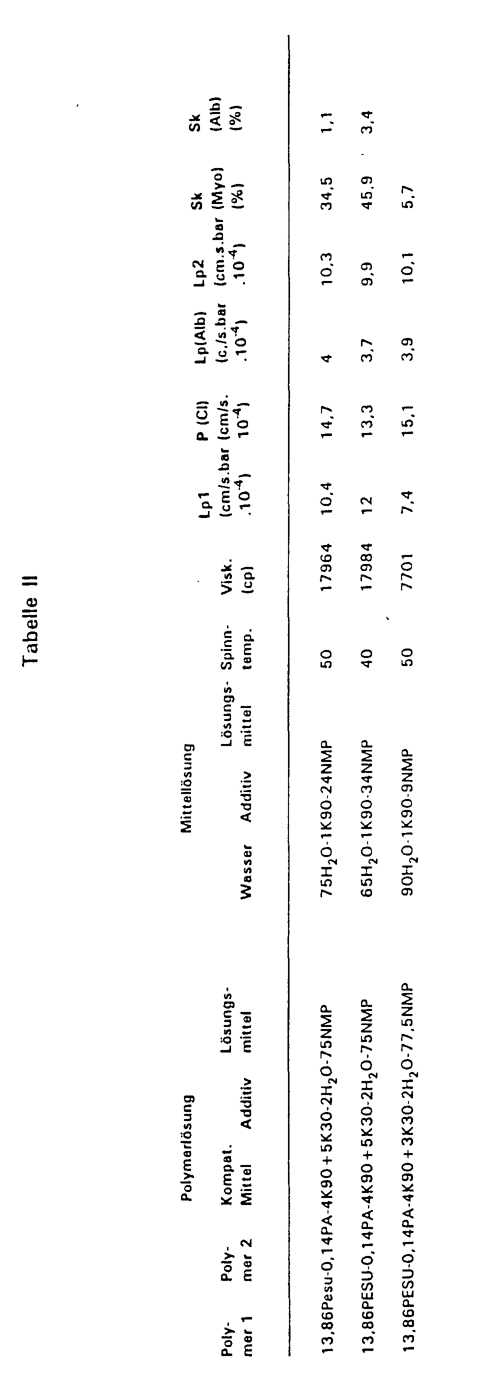

## Patentansprüche

1. Mit Hitze sterilisierbare Membran aus wenigstens zwei als solche nicht miteinander mischbaren hydrophoben Polymeren, die miteinander kompatibilisiert oder kovalent aneinander gebunden wurden, **dadurch gekennzeichnet**, daß unter den Bedingungen der Sterilisation mit gesättigtem Wasserdampf während 20 min bei 120 °C oder mit trockener Hitze von 180 °C während 90 min wenigstens eines der Polymeren als solches instabil und wenigstens eines der Polymeren als solches stabil ist.

2. Membran nach Anspruch 1, **dadurch gekennzeichnet,** daß sie aus 1 bis 30 Gew.-% des wenigstens einen unter den Bedingungen der Sterilisation mit Wasserdampf oder trockener Hitze instabilen Polymers und 70 bis 99 % des wenigstens einen unter den Bedingungen der Sterilisation mit Wasserdampf stabilen Polymers besteht.

3. Membran nach Anspruch 2, **dadurch gekennzeichnet,** daß sie aus 4 bis 12 Gew.-% des wenigstens einen unter den Bedingungen der Sterilisation mit Wasserdampf oder trockener Hitze instabilen Polymers und 88 bis 96 % des wenigstens einen unter den Bedingungen der Sterilisation mit Wasserdampf stabilen Polymers besteht.

4. Membran nach einem der Ansprüche 1 bis 3, **dadurch gekennzeichnet,** daß die unter den Bedingungen der Sterilisation mit Wasserdampf oder trockener Hitze stabilen Polymere eine Glasübergangstemperatur Tg >200 °C haben.

5. Membran nach einem der Ansprüche 1 bis 4, **dadurch gekennzeichnet,** daß das unter den Bedingungen der Sterilisation mit Wasserdampf oder trockener Hitze instabile Polymer ein teilaromatisches Polyamid, ein Polycarbonatether, ein Polyacrylnitril oder ein Cellulosetriacetat ist.

6. Membran nach Anspruch 5, **dadurch gekennzeichnet,** daß das unter den Bedingungen der Sterilisation mit Wasserdampf oder trockener Hitze instabile Polymer ein teilaromatisches amorphes Polyamid ist.

7. Membran nach einem der Anspirüche 1 bis 6, **dadurch gekennzeichnet,** daß das unter den Bedingungen der Sterilisation mit Wasserdampf oder trockener Hitze stabile Polymer ein Polyethersulfon, ein Polysulfon, ein Polyetherketon oder ein Polyaramid ist.

8. Membran nach Anspruch 7, **dadurch gekennzeichnet,** daß das unter den Bedingungen der Sterilisation mit Wasserdampf oder trockener Hitze stabile Polymer ein Polyethersulfon oder Polysulfon ist.

9. Membran nach einem der Ansprüche 1 bis 8, **dadurch gekennzeichnet,** daß ihre Polymeren in molekularer Verteilung oder bis zu einer Teilchengröße von maximal 1 µm vorliegen.

10. Membran nach einem der Ansprüche 1 bis 9, **dadurch gekennzeichnet,** daß die als solche nicht miteinander mischbaren hydrophoben Polymeren mit Hilfe eines amphiphilen Mittels kompatibilisiert sind.

11. Membran nach Anspruch 10, **dadurch gekennzeichnet,** daß sie das amphiphile Mittel in einer Menge von 5 bis 10 Gew.-% enthält.

12. Membran nach einem der Ansprüche 1 bis 9, **dadurch gekennzeichnet,** daß die als solche nicht miteinander mischbaren hydrophoben Polymeren durch Sulfonierung des wenigstens einen unter den Bedingungen der Sterilisation mit Wasserdampf oder trockener Hitze stabilen Polymers kompatibilisiert sind.

13. Membran nach einem der Ansprüche 1 bis 9, **dadurch gekennzeichnet,** daß die als solche nicht miteinander mischbaren hydrophoben Polymeren mit Hilfe von Polyvinylpyrrolidon miteinander kompatibilisiert sind.

## Claims

1. A heat-sterilisable membrane comprising at least two hydrophobic polymers which as such are not miscible with each other and which are compatibilised with each other or covalently bonded to each other characterised in that under the conditions of sterilisation with saturated steam for a period of 20 minutes at 120°C or with dry heat at 180°C for a period of 90 minutes at least one of the polymers as such is unstable and at least one of the polymers as such is stable.

2. A membrane according to claim 1 characterised in that it comprises from 1 to 30% by weight of the at least one polymer which is unstable under the conditions of sterilisation with steam or dry heat and from 70 to 99% of the at least one polymer which is stable under the conditions of sterilisation with steam.

3. A membrane according to claim 2 characterised in that it comprises from 4 to 12% by weight of the at least one polymer which is unstable under the conditions of sterilisation with steam or dry heat and from 88 to 96% of the at least one polymer which is stable under the conditions of sterilisation with steam.

4. A membrane according to one of claims 1 to 3 characterised in that the polymers which are stable under the conditions of sterilisation with steam or dry heat have a vitreous transition temperature Tg >200°C.

5. A membrane according to one of claims 1 to 4 characterised in that the polymer which is unstable under the conditions of sterilisation with steam or dry heat is a partially aromatic polyamide, a polycarbonate ether; a polyacrylonitrile or a cellulose triacetate.

6. A membrane according to claim 5 characterised in that the polymer which is unstable under the conditions of sterilisation with steam or dry heat is a partially aromatic amorphous polyamide.

7. A membrane according to one of claims 1 to 6 characterised in that the polymer which is stable under the conditions of sterilisation with steam or dry heat is a polyethersulphone, a polysulphone, a polyetherketone or a polyaramide.

8. A membrane according to claim 7 characterised in that the polymer which is stable under the conditions of sterilisation with steam or dry heat is a polyethersulphone or polysulphone.

9. A membrane according to one of claims 1 to 8 characterised in that its polymers are present in a molecular distribution or up to a particle size of a maximum of 1 µm.

10. A membrane according to one of claims 1 to 9 characterised in that the hydrophobic polymers which as such are not miscible with each other are compatibilised by means of an amphiphilic agent.

11. A membrane according to claim 10 characterised in that it contains the amphiphilic agent in an amount of from 5 to 10% by weight.

12. A membrane according to one of claims 1 to 9 characterised in that the hydrophobic polymers which as such are not miscible with each other are compatibilised by sulphonation of the at least one polymer which is stable under the conditions of sterilisation with steam or dry heat.

13. A membrane according to one of claims 1 to 9 characterised in that the hydrophobic polymers which as such are not miscible with each other are compatibilised with each other by means of polyvinylpyrrolidone.

## Revendications

1. Membrane stérilisable par la chaleur, constituée d'au moins deux polymères hydrophobes non miscibles l'un avec l'autre en tant que tels, qui ont été liés l'un à l'autre en étant rendus compatibles entre eux ou par covalence, caractérisée en ce que, dans les conditions de la stérilisation à l'aide de vapeur d'eau saturée durant 20 mn à 120°C ou à l'aide d'une chaleur sèche de 180°C durant 90 mn, l'un au moins des polymères, en tant que tel, est instable et l'un au moins des polymères, en tant que tel, est stable.

2. Membrane selon la revendication 1, caractérisée en ce qu'elle se compose de 1 à 30% en poids du ou des polymère(s) instable(s) dans les conditions de la stérilisation à l'aide de vapeur d'eau ou de chaleur sèche et de 70% à 99% du ou des polymère(s) stable(s) dans les conditions de la stérilisation à l'aide de vapeur d'eau.

3. Membrane selon la revendication 2, caractérisée en ce qu'elle se compose de 4 à 12% en poids du ou des polymère(s) instable(s) dans les conditions de la stérilisation à l'aide de vapeur d'eau ou de chaleur sèche et de 88% à 96% du ou des polymère(s) stable(s) dans les conditions de la stérilisation à l'aide de vapeur d'eau.

4. Membrane selon l'une des revendications 1 à 3, caractérisée en ce que les polymères stables dans les conditions de la stérilisation à l'aide de vapeur d'eau ou de chaleur sèche ont une température de transition vitreuse Tg > 200°C.

5. Membrane selon l'une des revendications 1 à 4, caractérisée en ce que le polymère instable dans les conditions de la stérilisation à l'aide de vapeur d'eau ou de chaleur sèche est un polyamide partiellement aromatique, un polycarbonate éther, un polyacrylonitrile ou un triacétate de cellulose.

6. Membrane selon la revendication 5, caractérisée en ce que le polymère instable dans les conditions de la stérilisation à l'aide de vapeur d'eau ou de chaleur sèche est un polyamide amorphe partiellement aromatique.

7. Membrane selon l'une des revendications 1 à 6, caractérisée en ce que le polymère stable dans les conditions de la stérilisation à l'aide de vapeur d'eau ou de chaleur sèche est un polyéther-sulfone, un polysulfone, un polyéther-cétone ou un polyaramide.

8. Membrane selon la revendication 7, caractérisée en ce que le polymère stable dans les conditions de la stérilisation à l'aide de vapeur d'eau ou de chaleur sèche est un polyéther-sulfone ou un polysulfone.

9. Membrane selon l'une des revendications 1 à 8, caractérisée en ce que ses polymères sont présents suivant une distribution moléculaire ou jusqu'à une taille de particules de 1 µm au maximum.

10. Membrane selon l'une des revendications 1 à 9, caractérisée en ce que les polymères hydrophobes, non miscibles l'un avec l'autre en tant que tels, sont rendus compatibles au moyen d'un agent amphiphile.

11. Membrane selon la revendication 10, caractérisée en ce qu'elle contient l'agent amphiphile en une proportion de 5 à 10% en poids.

12. Membrane selon l'une des revendications 1 à 9, caractérisée en ce que les polymères hydrophobes, non miscibles l'un avec l'autre en tant que tels, sont rendus compatibles par sulfonation du ou des polymère(s) stable(s) dans les conditions de la stérilisation à l'aide de vapeur d'eau ou de chaleur sèche.

13. Membrane selon l'une des revendications 1 à 9, caractérisée en ce que les polymères hydrophobes, non miscibles l'un avec l'autre en tant que tels, sont rendus compatibles entre eux au moyen de polyvinylpyrrolidone.
